# FASCICULE DE BREVET EUROPEEN

(11) **EP 2 381 927 B1**
(45) Date de publication et mention de la délivrance du brevet: **21.09.2016**
(21) Numéro de dépôt: 09804194.0
(22) Date de dépôt: 24.12.2009
(51) Int. Cl.: A61K 9/14, A61K 9/16, A61K 9/20, A61K 9/50, A61K 31/216

(54) **FORMULATION PHARMACEUTIQUE DE FENOFIBRATE NANONISE**
PHARMAZEUTISCHE FORMULIERUNG VON NANONISIERTEM FENOFIBRAT
PHARMACEUTICAL FORMULATION OF NANONISED FENOFIBRATE

(30) Priorité: 24.12.2008 FR 0859064
(43) Date de publication de la demande: 02.11.2011
(73) Titulaire: ETHYPHARM, 92210 Saint-Cloud (FR)
(72) Inventeur: HERRY, Catherine, 27670 Saint-Ouen du Tilleul (FR); OURY, Pascal, F-78150 Le Chesnay (FR)
(74) Mandataire: Regimbeau
(86) Numéro de dépôt international: PCT/EP2009/067915
(87) Numéro de publication internationale: WO 2010/081623

(56) Documents cités:
- EP-A- 1 574 214
- WO-A-03/092659
- WO-A-2005/032526
- WO-A-2008/016260
- US-A1- 2008 138 424

## Description

La présente invention a pour objet une nouvelle composition pharmaceutique contenant du fénofibrate ainsi qu'un procédé de préparation d'une suspension aqueuse contenant du fénofibrate et/ou de l'acide fénofibrique.

Le fénofibrate, également connu comme 2-[4-(4-chlorobenzoyl)phénoxy]-2-méthyl-propanoate de 1-méthyléthyle, est un agent régulateur des lipides. Il présente un poids moléculaire de 360,83 g/mol et un point de fusion à l'état cristallin compris entre 79 et 82°C.

Le fénofibrate est préconisé dans le traitement des hyperlipidémies, des hypercholestérolémies et des hypertriglycéridémies endogènes de l'adulte. Un traitement de 300 à 400 mg de fénofibrate par jour permet une réduction de 20 à 25 % de la cholestérolémie et de 40 à 50 % de la triglycéridémie.

Le métabolite majeur plasmatique du fénofibrate est l'acide fénofibrique qui est la forme active du fénofibrate. Le point de fusion à l'état cristallin de l'acide fénofibrique est compris entre 179 et 183°C. La demi-vie plasmatique d'élimination de l'acide fénofibrique est de l'ordre de 20 heures. Sa concentration plasmatique maximale est atteinte en moyenne cinq heures après l'ingestion du médicament. La concentration plasmatique moyenne est de l'ordre de 15 microgrammes/ ml pour une posologie de 300 mg de fénofibrate par jour. Ce taux est stable tout au long du traitement.

Le fénofibrate est un principe actif très faiblement soluble dans l'eau dont l'absorption au niveau du tractus digestif est limitée. Une augmentation de sa solubilité ou de sa vitesse de solubilisation entraîne une meilleure absorption digestive.

A cause de cette faible affinité pour l'eau et sa nature hydrophobe, le fénofibrate est mieux absorbé après l'ingestion d'aliments que dans des conditions à jeun. Ce phénomène est appelé le « food effect ». Il est particulièrement important quand l'on compare l'absorption de fénofibrate dans des conditions de prise d'un repas gras versus des conditions à jeun.

Le principal inconvénient de ce « food effect » est que le régime alimentaire du patient doit être contrôlé pendant le traitement. En outre, comme le fénofibrate est mieux absorbé pendant un repas gras, il est habituellement pris après un repas gras, ce qui est incompatible avec un traitement des hyperlipidémies, des hypercholestérolémies et des hypertriglycéridémies.

Diverses voies ont été explorées pour augmenter la vitesse de solubilisation du fénofibrate : la micronisation du principe actif, l'ajout d'un tensioactif, la co-micronisation du fénofibrate avec un tensioactif, la sub-micronisation du fénofibrate.

Le brevet EP 256 933 décrit des granules de fénofibrate dans lesquels le fénofibrate est micronisé pour augmenter sa biodisponibilité. Les microparticules cristallines de fénofibrate sont de dimension inférieure à 50 µm. Le liant utilisé est la polyvinylpyrrolidone. Le document suggère d'autres types de liants comme les polymères métacryliques, les dérivés de cellulose, et les polyéthylènes glycols. Les granules décrits dans les exemples de EP 256 933 sont obtenus par un procédé mettant en oeuvre des solvants organiques.

Le brevet EP 330 532 propose d'améliorer la biodisponibilité du fénofibrate en le co-micronisant avec un tensioactif, comme le laurylsulfate de sodium. Le co-micronisat est ensuite granulé par voie humide afin d'améliorer les capacités d'écoulement de la poudre et de faciliter la mise en gélules. Cette co-micronisation permet une augmentation significative de la biodisponibilité par rapport à l'utilisation de fénofibrate décrite dans EP 256 933. Les granulés décrits dans EP 330 532 contiennent de la polyvinylpyrrolidone comme liant.

Ce brevet enseigne que la co-micronisation du fénofibrate avec un tensioactif solide améliore significativement la biodisponibilité du fénofibrate comparativement à l'utilisation d'un tensioactif, d'une micronisation ou de l'association d'un tensioactif et du fénofibrate micronisé.

Le brevet WO 98/31361 propose d'améliorer la biodisponibilité du fénofibrate, en fixant sur un support inerte hydrodispersible du fénofibrate micronisé, un polymère hydrophile et éventuellement un tensioactif. Le polymère hydrophile, identifié comme de la polyvinylpyrrolidone, représente au moins 20 % en poids de la composition précédemment décrite.

Ce procédé permet d'augmenter la vitesse de dissolution du fénofibrate, ainsi que sa biodisponibilité. Cependant, le procédé de préparation selon ce brevet n'est pas totalement satisfaisant, car il nécessite la mise en oeuvre d'une quantité importante de PVP et des autres excipients. L'exemple présenté dans cette demande de brevet, fait part d'une composition contenant seulement 17,7 % de fénofibrate exprimé en rapport massique. Ce faible rapport massique du fénofibrate entraîne une forme finale de très grande taille d'où une administration non aisée de la dose souhaitée de fénofibrate, ou l'administration de deux comprimés.

Les demandes WO2004/041250 et US2008/138424 concernent des compositions de fibrate comprenant des particules de fibrate de taille moyenne sub-micronique inférieure à 2000 nm.

La nanonisation du fénofibrate permet encore d'améliorer son profil de dissolution. Toutefois, plusieurs inconvénients résultent de cette diminution de taille. Les particules nanonisées ne sont pas stables et tendent à se réagglomérer. D'autre part, le broyage à des dimensions de l'ordre du nanomètre présente un risque élevé de changement de forme cristalline du principe actif de la forme polymorphe à la forme amorphe. Or, la forme amorphe est moins stable que la forme cristalline et évoluera vers la forme cristalline au cours du temps avec un impact potentiel sur le comportement en dissolution et chez l'homme.

Il a été découvert dans le cadre de la présente invention que les conditions du broyage liquide permettant d'obtenir du fénofibrate nanonisé peuvent être optimisées afin de maintenir le fénofibrate sous forme cristalline pendant le broyage et d'obtenir une biodisponibilité et une stabilité supérieures tout en conservant un « food effect » nul.

La présente invention a donc pour objet le procédé de préparation d'une suspension aqueuse contenant du fénofibrate et/ou de l'acide fénofibrique en suspension, un dérivé cellulosique en tant qu'adjuvant de solubilisation et un tensioactif, le fénofibrate et/ou l'acide fénofibrique étant sous forme cristalline de point de fusion en calorimétrie différentielle à balayage (DSC) respectivement de 79°C à 82°C et de 179°C à 183°C et la taille moyenne des particules de fénofibrate et/ou d'acide fénofibrique (D(50)) étant inférieure à 250 nm, de préférence 180 nm. La calorimétrie différentielle à balayage est utilisée pour caractériser les températures de fusion ou de cristallisation de solides cristallins, ou les températures de transition vitreuse de solides amorphes.

De préférence, la suspension aqueuse obtenue par le procédé selon l'invention comprend du fénofibrate ou de l'acide fénofibrique.

De préférence, 90% des particules (D(90)) ont une taille inférieure à 500 nm, de préférence, 400 nm. La taille des particules est déterminée par granulométrie en diffraction laser ou en diffusion de la lumière.

Avantageusement, la suspension obtenue par le procédé selon l'invention comprend en outre un phospholipide, préférentiellement la lécithine de soja.

L'ajout d'un phospholipide à la suspension améliore la stabilité de la suspension. Au cours du temps, la suspension sédimente. En l'absence de phospholipide, sa mise en suspension après agitation est difficile voire impossible : la phase solide de la suspension forme un gâteau difficile à disperser. Lorsqu'un phospholipide est ajouté à la suspension, une simple agitation permet de remettre en suspension la phase solide. Le phospholipide présente également l'avantage de permettre la formation de micelles dans l'eau, favorisant la mise à disposition du fénofibrate pour absorption.

Avantageusement, la proportion de phospholipide dans la suspension obtenue par le procédé de l'invention est comprise entre 0,25% et 10%, de préférence entre 0,3 et 5%, de préférence encore entre 0,5 et 1% en poids de la suspension.

Avantageusement, la proportion totale de fénofibrate et d'acide fénofibrique dans la suspension obtenue par le procédé de l'invention est comprise entre 10% et 20%, de préférence entre 12 et 17%, en poids de la suspension.

Le dérivé cellulosique liant représente entre 0,1 et 10 %, de préférence entre 1 et 5 % en poids de la composition.

Avantageusement, le dérivé cellulosique est l'hydroxypropylméthylcellulose.

On choisit de préférence l'hydroxypropylméthylcellulose dont la viscosité apparente est comprise entre 3 et 15 mPa.s et de manière encore plus préférée comprise entre 3 et 6 mPa.s, comme par exemple le Pharmacoat 603®.

Le tensioactif est choisi parmi les tensioactifs solides ou liquides à température ambiante, par exemple le laurylsulfate de sodium, le Polysorbate 80 ou le Montane 20, de préférence le laurylsulfate de sodium.

Le rapport fénofibrate et/ou acide fénofibrique /HPMC est de préférence compris entre 5/1 et 15/1.

L'agent tensioactif représente entre 0,1 à 3 %, de préférence entre 0,3 et 1 % en poids par rapport au poids de la suspension.

La suspension obtenue par le procédé de l'invention peut en outre contenir au moins un excipient tel que des agents anti-mousse comme la diméthicone et la siméthicone.

La présente invention concerneun procédé de préparation de la suspension selon l'invention, comprenant les étapes suivantes :
(a) mélanger une partie de la proportion de dérivé cellulosique dans la suspension, le tensioactif et éventuellement le phospholipide avec de l'eau sous agitation jusqu'à complète dispersion,
(b) ajouter le fénofibrate et/ou l'acide fénofibrique au mélange obtenu à l'étape précédente sous agitation jusqu'à complète dispersion, puis ;
(c) broyer le mélange obtenu à l'étape précédente en présence de billes à une puissance suffisante pour atteindre une granulométrie moyenne du fénofibrate (D(50)) inférieure à 250 nm ;
(d) éventuellement homogénéiser la suspension broyée obtenue à l'étape précédente ;
(e) ajuster la quantité de dérivé cellulosique et éventuellement d'eau.

Ce procédé de préparation de la suspension consistant à ajouter une partie du dérivé cellulosique avant le broyage et le reste après le broyage est avantageux. En effet, il permet de limiter la viscosité de la suspension et d'améliorer l'efficacité du broyage.

La proportion de dérivé cellulosique mélangée à l'étape (a) est avantageusement comprise entre 20 et 40%, préférentiellement d'environ 30%, de la quantité de dérivé cellulosique dans la suspension finale.

L'étape (a) peut être décomposée en une étape d'ajout du dérivé cellulosique et agitation jusqu'à dissolution complète, une étape d'ajout du tensioactif et agitation jusqu'à dissolution complète, et éventuellement une étape d'ajout du phospholipide et agitation jusqu'à dispersion complète. Ces étapes peuvent s'enchaîner dans n'importe quel ordre. Préférentiellement, le dérivé cellulosique est ajouté avant le tensioactif.

Le broyage est réalisé en présence de billes. Elles sont préférentiellement en polymère, avantageusement en polystyrène, ou en oxyde de zirconium et/ou d'yttrium. Leur diamètre est compris entre 0,1 et 0,8 mm, préférentiellement il est compris entre 0,2 mm et 0,6 mm.

La chambre de broyage est remplie à au moins 70% par les billes, préférentiellement au moins 80%. La température du produit pendant le broyage est maintenue en dessous de 35°C, préférentiellement 30°C. Avantageusement, la distribution de taille des particules est mesurée en granulométrie en diffraction laser ou en diffusion de la lumière chaque heure et le broyage est stoppé quand le diamètre ne diminue plus.

Le procédé de préparation d'une suspension de l'invention peut comprendre une ou plusieurs étapes d'ajout d'agent anti-mousse, avantageusement la diméthicone et/ou la siméthicone. Cette ou ces étape(s) peuvent intervenir avant ou après le broyage. Préférentiellement, au moins une étape d'ajout d'agent anti-mousse est réalisée avant le broyage pour limiter voire éviter la formation de mousse pendant le broyage.

La présente invention concerne également un procédé de préparation de microgranules ou de granules de fénofibrate et/ou d'acide fénofibrique comprenant une ou plusieurs couches de la suspension obtenue par le procédé selon l'invention, avantageusement deux couches.

De préférence, les microgranules ou granules selon l'invention comprennent du fénofibrate ou de l'acide fénofibrique.

On appelle microgranules des supports sphériques sur lesquelles une suspension d'actif peut être pulvérisée. Ils peuvent être de toute nature chimique et préférentiellement à base de sucre et amidon, de cellulose, ou d'aluminosilicate de magnésium.

On appelle granules des particules de formes plus ou moins irrégulières obtenues par pulvérisation d'un principe actif sur un excipient à l'état de poudre plus ou moins fine ou par un procédé de granulation humide.

Selon une première variante, les microgranules ou granules de fénofibrate et/ou d'acide fénofibrique selon l'invention sont préparés par un ou plusieurs montages, avantageusement deux, de la suspension obtenue par le procédé de l'invention sur des neutres.

Les microgranules neutres ont une granulométrie comprise entre 200 et 1000 microns, de préférence entre 400 et 600 microns.

Le montage est effectué en turbine à dragéification, en turbine perforée ou en lit fluidisé, de préférence en lit fluidisé.

Le montage sur des microgranules neutres peut être réalisé par pulvérisation d'une suspension aqueuse selon l'invention.

Les microgranules de l'invention montés sur des neutres sont particulièrement appropriés à la préparation de gélules.

Les microgranules de l'invention montés sur des neutres comprennent avantageusement une étape de surenrobage après la ou les étapes de montage, et préférentiellement une étape de lubrification après l'étape de surenrobage.

La couche de surenrobage est préférentiellement constituée d'un dérivé cellulosique, avantageusement d'HPMC. La proportion de dérivé cellulosique de surenrobage dans la composition de surenrobage est d'au moins 3%.

Le lubrifiant préféré est le talc.

Selon une deuxième variante, les granules de fénofibrate et/ou d'acide fénofibrique selon l'invention sont préparés par montage sur des granules d'excipient.

Des excipients appropriés comprennent les sucres, préférentiellement les lactoses, la cellulose microcristalline, par exemple l'Avicel PH200.

Les excipients utilisés sont préférentiellement les excipients connus de l'homme du métier pour présenter de bonnes propriétés de compression, soit les excipients utilisés pour préparer des granules destinés à être comprimés.

La deuxième variante de préparation de granules de fénofibrate et/ou d'acide fénofibrique selon l'invention est donc particulièrement adaptée à la préparation de granules destinés à la préparation de comprimés.

Un autre objet de l'invention concerne des microgranules ou granules de fénofibrate et/ou d'acide fénofibrique susceptibles d'être obtenus selon le procédé de préparation de microgranules ou granules de fénofibrate et/ou d'acide fénofibrique de l'invention.

Les microgranules ou granules de fénofibrate et/ou d'acide fénofibrique selon l'invention comprennent avantageusement entre 1% et 10%, de préférence entre 3 et 6% en poids de phospholipide.

Les microgranules ou granules de fénofibrate et/ou d'acide fénofibrique selon l'invention comprennent avantageusement plus de 60% en poids de fénofibrate.

Les microgranules ou granules de fénofibrate et/ou d'acide fénofibrique selon l'invention comprennent avantageusement entre 1% et 10%, de préférence entre 3 et 5% en poids de tensioactif.

Les microgranules ou granules de fénofibrate et/ou d'acide fénofibrique selon l'invention comprennent avantageusement entre 7% et 20%, de préférence entre 10 et 15% en poids de dérivé cellulosique, avantageusement d'hydroxypropylméthylcellulose, en tant que liant et adjuvant de solubilisation.

Les microgranules ou granules de fénofibrate et/ou d'acide fénofibrique selon l'invention comprennent avantageusement entre 0% et 5%, de préférence entre 1 et 3% en poids de lubrifiant.

Les microgranules ou granules de fénofibrate et/ou d'acide fénofibrique selon l'invention comprennent avantageusement entre 0% et 7%, de préférence entre 0 et 3% en poids d'agent anti-mousse.

Les microgranules de fénofibrate et/ou d'acide fénofibrique selon l'invention comprennent avantageusement de l'intérieur vers l'extérieur :
- un coeur constitué d'un neutre,
- deux couches d'enrobage constituées de la suspension selon l'invention
- une couche de surenrobage, avantageusement comprenant de l'HPMC,
- une couche de lubrifiant, avantageusement de talc.

Les granules de fénofibrate et/ou d'acide fénofibrique comprennent avantageusement, comprenant de l'intérieur vers l'extérieur :
- un coeur constitué d'un excipient, avantageusement de lactose directement compressible comme, par exemple, le Lactose DCL21,
- une couche d'enrobage constituée de la suspension selon l'invention.

Un autre objet de l'invention concerne une formulation pharmaceutique unitaire solide comprenant des microgranules ou granules de fénofibrate et/ou d'acide fénofibrique selon l'invention.

De préférence, la formulation pharmaceutique unitaire solide selon l'invention comprend des microgranules ou granules de fénofibrate ou d'acide fénofibrique selon l'invention.

La formulation pharmaceutique de l'invention comprend avantageusement des microgranules ou granules de fénofibrate selon l'invention en une quantité équivalant à une dose de fénofibrate comprise entre 20 et 200 mg, de préférence comprise entre 110 et 145 mg, de façon particulièrement préférée entre 110 et 120 mg ou entre 30 et 40 mg, par exemple égale à 115, 120, 130 ou 145 mg.

La formulation pharmaceutique de l'invention comprenant des microgranules ou granules de fénofibrate selon l'invention présente avantageusement les paramètres pharmacocinétiques suivants :
(a) AUC∞ comprise entre 105000 et 180000 ng.h/mL et
(b) Cmax compris entre 6500 et 12000 ng/mL.
avec Cmax signifie la concentration plasmatique maximale exprimée en ng/mL, et AUC∞ signifie l'aire sous la courbe de 0 à l'infini, exprimée en ng.h/mL

La formulation pharmaceutique de l'invention comprend avantageusement des microgranules ou granules d'acide fénofibrique selon l'invention en une quantité équivalant à une dose d'acide fénofibrique comprise entre 18 et 180 mg, de préférence égale à 135 mg.

La formulation pharmaceutique de l'invention se présente sous forme de gélules, de comprimés orodispersibles ou de comprimés.

Pour la forme gélule, les microgranules de fénofibrate et/ou d'acide fénofibrique sont préparés selon la première variante. La suspension selon l'invention est montée sur des neutres, en une ou plusieurs couches, puis les microgranules sont recouverts d'une couche externe de surenrobage avant leur lubrification.

Pour la forme comprimé, les granules de fénofibrate et/ou d'acide fénofibrique de l'invention sont préparés selon la seconde variante. La suspension selon l'invention est montée sur des granules d'excipient puis les granules sont mélangés à des adjuvants de compression comme des agents désintégrants, diluants, liants, lubrifiants.

Parmi les agents de désintégration, sont préférés les dérivés réticulés de la carboxymethylcellulose, les dérivés de la polyvinylpyrrolidone, les dérivés de l'amidon.

Parmi les diluants, on retrouve les sucres, préférentiellement les lactoses, les dérivés cellulosiques, notamment la cellulose microcristalline.

Parmi les lubrifiants, le stéarate de magnésium et le stéarylfumarate de sodium sont préférés.

Un autre objet de l'invention concerne une formulation pharmaceutique de fénofibrate et/ou d'acide fénofibrique selon l'invention pour le traitement des hypertriglycéridémies, hypercholestérolémies ou hyperlipidémies.

Avantageusement ce traitement présente un profil pharmacocinétique du fénofibrate et/ou d'acide fénofibrique équivalent que le patient soit nourri avec un repas à forte teneur en matières grasses ou à jeun.

Un autre objet de l'invention concerne l'utilisation des microgranules ou granules selon l'invention pour la fabrication d'un médicament destiné au traitement des hypertriglycéridémies, hypercholestérolémies ou hyperlipidémies.

### Figures

La figure 1 représente le profil de dissolution des lots 3 montages (D05274) et 2 montages (D05291) de la formulation de l'exemple 2C.
La figure 2 représente le profil de dissolution deux montages en fonction du temps en min, la température de broyage étant inférieure à 30°C. (Exemple 2D)
La figure 3 représente le profil de dissolution des gélules de l'Exemple 2E (D05300) versus Antara® et Tricor®.
La figure 4 montre les diffractogrammes du fénofibrate (en haut), du lactose anhydre (au milieu) et des granules montés sur lactose anhydre (en bas). (Exemple 3)
La figure 5 montre les diffractogrammes des granules (en haut), et de deux lots de comprimés (au milieu et en bas). Tous les pics des comprimés peuvent être attribués au fénofibrate ou au lactose anhydre. Ils ne comprennent donc pas de fénofibrate en phase amorphe. (Exemple 3)
La figure 6 montre l'évolution du diamètre moyen des particules de fénofibrate mesuré en nm (échelle de gauche) en fonction de la viscosité (échelle de droite) mesurée en cP et du temps en min (en abscisse) (Exemple 1).
La figure 7 montre le profil de libération *in vivo* d'une formulation de fénofibrate nanonisé sous forme de gélule comprenant 0,3% de lécithine de soja dans la suspension (Exemple 4).
La figure 8 montre le profil de libération *in vivo* des comprimés 145 mg de fénofibrate nanonisé selon l'invention (TEST) et celui du Lipanthyl® (REFERENCE) chez le sujet à jeun.
La figure 9 montre le profil de libération in vivo des comprimés 145 mg de fénofibrate nanonisé selon l'invention (TEST) et celui du Lipidil EZ® (REFERENCE) chez le sujet à jeun.

L'invention est illustrée par les exemples suivants.

### Exemple 1: Préparation d'une suspension de fénofibrate nanonisé

La formule de la suspension est la suivante:

| LOT | **MASSE DU LOT (G)** | **FORMULE CENTÉSIMALE** |
|---|---|---|
| **Fénofibrate** | 437.3 | 17.49 |
| **HPMC** | 82.7 | 3.31 |
| **Diméthicone 35%** | 4.8 | 0.19 |
| **Siméthicone 30%** | 0.8 | 0.03 |
| **Laurylsulfate de Sodium** | 22.3 | 0.89 |
| **Eau purifiée** | 1952.2 | 78.08 |
| **Total** | 2500.1 | 100.00 |

Le broyage est effectué dans un broyeur en voie humide Netzsch Labstar LS1 chargé à 88% de billes de ZrO₂ Y₂O₃ de diamètre 0,3mm. Les paramètres de broyage sont présentés ci-dessous

| **Billes** | Oxyde de zirconium 0.3 mm, 88% |
|---|---|
| **Vitesse du broyeur (rpm)** | 3000 i.e. 7.9 m/s |
| **Température du Produit (°C)** | 39 |
| **Energie (kWh)** | 9.8 |

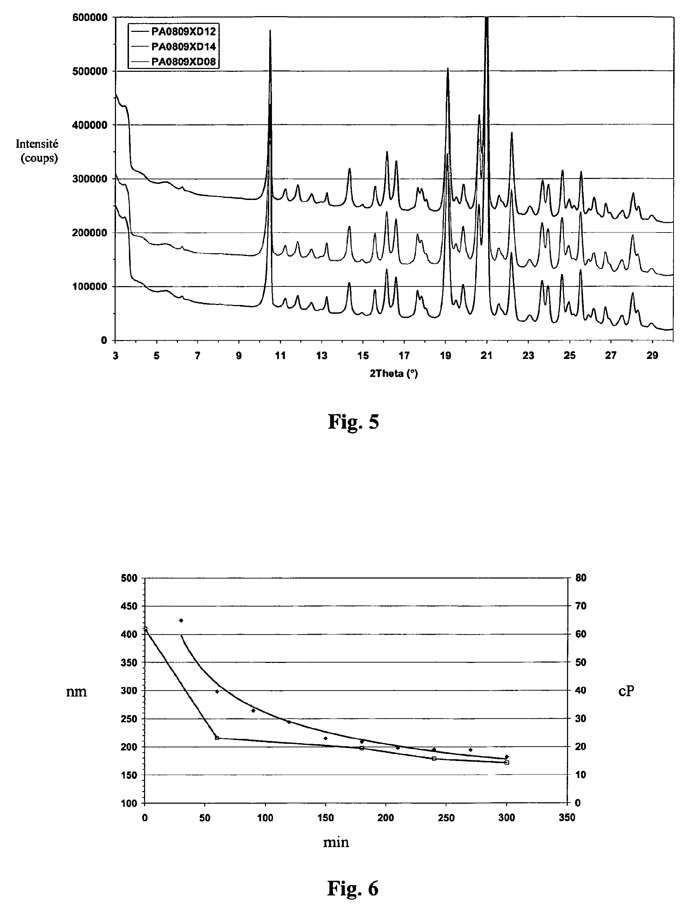

Le diamètre moyen du fénofibrate après 5h de broyage est de 180nm. La viscosité de la suspension diminue pendant le procédé, témoin de l'altération des chaînes d'HPMC (voir figure 6).

En l'état, cette suspension n'a pas un pouvoir suffisamment liant pour pouvoir être pulvérisée sur des granules pour obtenir une teneur acceptable.

Le retrait d'une partie d'HPMC permet d'avoir une suspension moins visqueuse, dont la viscosité n'évolue pas au cours du broyage. L'HPMC résiduelle est ajoutée après broyage ce qui permet de conserver son pouvoir liant.

### Exemple 2 : Préparation d'une formulation de fénofibrate nanonisé sous forme de gélule

### A- Formule de la suspension

### 1. Formule

La formule des suspensions de montage et les informations sur les matières premières utilisées sont présentées dans le tableau 1. Trois suspensions sont successivement broyées. Une partie de l'HPMC (30%) est introduite avant le broyage. Les 70% restants sont ajoutés après broyage.

**Tableau 1 - Formule des suspensions de montage**

| **MATIERE** | **FORMULE (G)** | **FORMULE CENTESIMALE (%)** | |
|---|---|---|---|
| **AVANT BROYAGE** | | | |
| Eau purifiée | 1376,60 | 74,49 | |
| Emulsion de siméthicone à 30% | 0,80 | 0,04 | |
| Emulsion de diméthicone à 35% | 4,70 | 0,25 | |
| Lauryl sulfate de sodium | 22,00 | 1,19 | |
| HPMC 603 | 24,00 | 1,30 | |
| Fénofibrate | 420,00 | 22,73 | |
| Total | | 1848,10 | 100,00 |
| **APRES BROYAGE** | | | |
| Suspension broyée | Eau purifiée | 1288,63 | 76,89 |
| | Emulsion de siméthicone à 30% | 0,75 | |
| | Emulsion de diméthicone à 35% | 4,40 | |
| | Lauryl sulfate de sodium | 20,59 | |
| | HPMC 603 | 22,47 | |
| | Fénofibrate | 393,16 | |
| | **Total suspension broyée** | **1730,00** | |
| Eau purifiée | | 468,00 | 20,80 |
| HPMC 603 | | 52,00 | 2,31 |
| Total | | 2250,00 | 100,00 |
| Quantité utilisée pour le montage | | 2216,00 | 98,49 |

### 2. Résultats granulométriques

Les suspensions de fénofibrate ont été analysées par mesure de la granulométrie après broyage.

Les résultats sont repris dans le tableau 4.

**Tableau 2 - Résultats granulométriques**

| **DETERMINATION** | **METHODES** | **SPECIFICATIONS** | **RESULTATS** |
|---|---|---|---|
| **Granulométrie** | | | |
| Suspension broyée n°1 | Coulter N4+ mode unimodal | < 300 n m | 279 nm |
| Suspension broyée n°2 | | < 300 n m | 293 nm |
| Suspension broyée n°3 | | < 300 n m | 298 nm |

### B- Préparation de la suspension

Le broyage de la suspension de fénofibrate est effectué avec le broyeur Netzsch Labstar LS1 équipé de billes de ZrO₂ Y₂O₃ de 0,2 mm de diamètre.

Le volume utile de la chambre de broyage est de 0,57 litres. La chambre de broyage a été remplie à 90% par les billes.

Les principaux paramètres de broyage sont résumés dans le tableau 2.

**Tableau 3 - Paramètres de broyage**

| | **BROYAGE N°1** | **BROYAGE N°2** | **BROYAGE N°3** |
|---|---|---|---|
| **VITESSE DU BROYEUR (tours/min)** | 2200 - 2400 | 2200 - 2500 | 2200 - 2300 |
| **DIAMETRE MOYEN EN FIN DE BROYAGE (nm)** | 279 | 293 | 298 |

### C- Préparation de microgranules. subissant 2 versus 3 broyages-montages

La suspension de l'étape A est montée sur neutres.

On procède à deux ou trois broyages-montages successifs pour obtenir la teneur souhaitée. Les microgranules obtenus sont lubrifiés avec du talc.

La formule théorique des granules est indiquée dans le tableau 4.

**Tableau 4 - Formule théorique des granules de fénofibrate**

| | **LOT 3 MONTAGES** | | **LOT 2 MONTAGES** | |
|---|---|---|---|---|
| **MATIERE** | **QUANTITE S (G)** | **FORMULE CENTESIMALE (%)** | **QUANTITE S (G)** | **FORMULE CENTESIMALE (%)** |
| Neutres 30 | 300,00 | 23,59 | 300,00 | 38,14 |
| Emulsion de siméthicone 30% | 0,44 | 0,03 | 0,22 | 0,03 |
| Emulsion de diméthicone 35% | 3,03 | 0,24 | 1,52 | 0,19 |
| Lauryl sulfate de sodium | 40,57 | 3,19 | 20,28 | 2,58 |
| HPMC 603 | 146,68 | 11,54 | 73,34 | 9,33 |
| Fénofibrate | 774,44 | 60,91 | 387,22 | 49,23 |
| Talc | 6,33 | 0,50 | 3,91 | 0,50 |
| **Total** | 1271,49 | 100,00 | 786,49 | 100,00 |

Le montage de la suspension de fénofibrate est effectué en lit fluidisé GPCG1.

La lubrification est faite en turbine conventionnelle avec 0,5% de talc par rapport à la masse de granules produits.

Les principaux paramètres de montage et de séchage sont résumés dans le tableau 5.

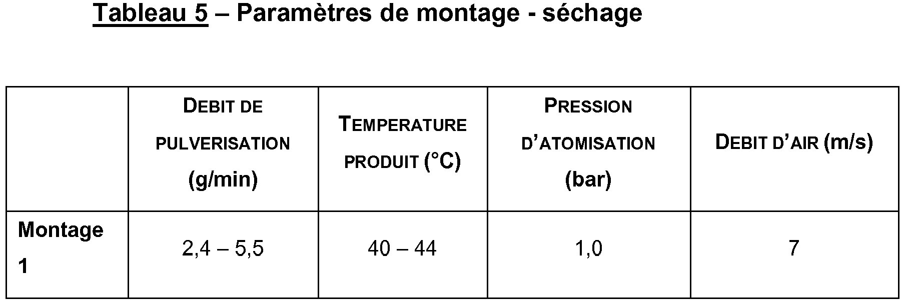

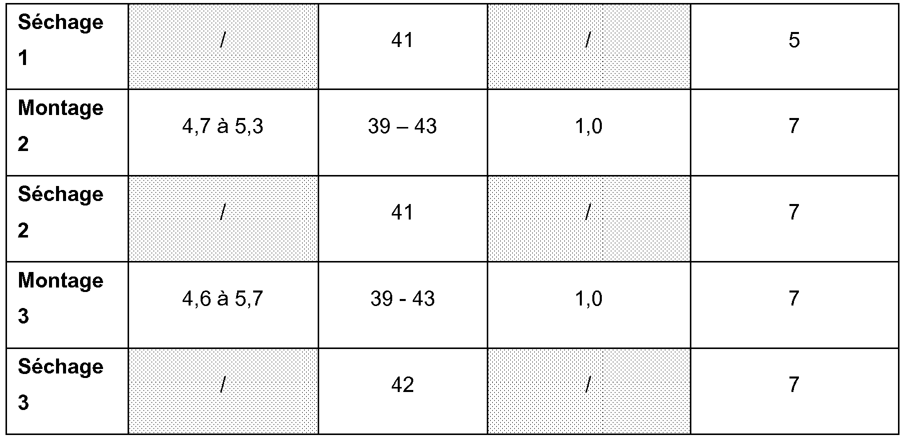

Les principaux résultats analytiques sont résumés dans le tableau 6.

**Tableau 6 - Masses, rendements, titres et profils de dissolution des granulés**

| **LOT** | **3 montages** | **2 montages** |
|---|---|---|
| **MICROGRANULES** | | |
| **RENDEMENT EN MASSE** | 93,0% | 91,7% |
| **TITRE THEORIQUE (mg/g)** | 609,08 | 492,34 |
| **Dissolution (voir** **figure 1****)** | 5 min: 50,7% | 5 min : 75,1% |
| | 10 min : 72,8% | 10 min: 92,7% |
| | 15 min : 81,7% | 15 min: 99,2% |
| | 30 min : 95,1% | 30 min : 102,7% |
| | 45 min: 98,4% | 45 min: 102,7% |
| | 60 min: 98,5% | 60 min: 102,4% |

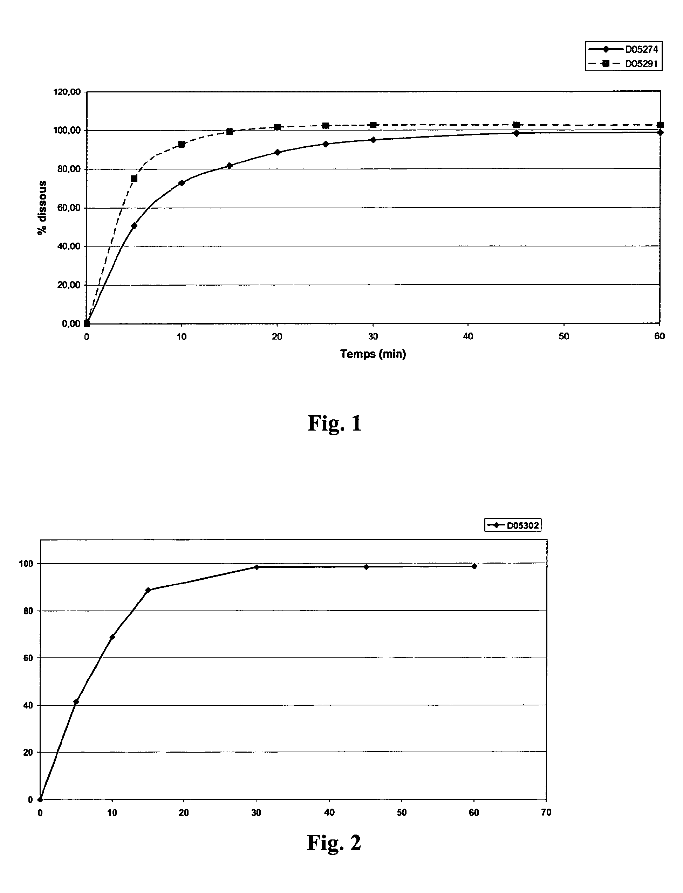

On peut observer sur la figure 1 que le profil de dissolution est plus rapide pour les granules présentant deux couches que pour les granules présentant trois couches.

### D- Préparation de microgranules subissant 2 versus 4 broyages-montages

La préparation des suspensions de broyage se fait de la même façon que précédemment : les billes utilisées sont de diamètre 0,2 mm. La température en cours de broyage est maintenue inférieure à 30°C.

La formule théorique des granulés est indiquée dans le tableau 7.

**Tableau 7 - Formule théorique des granules de fénofibrate**

| | **LOT 4 MONTAGES** | | **LOT 2 MONTAGES** | |
|---|---|---|---|---|
| **MATIERE** | **QUANTITES (G)** | **FORMULE CENTESIMALE (%)** | **QUANTITES (G)** | **FORMULE CENTESIMALE (%)** |
| Neutres 30 | 300,00 | 17,08 | 300,00 | 38,14 |
| Emulsion de siméthicone 30% | 0,66 | 0,04 | 0,22 | 0,03 |
| Emulsion de diméthicone 35% | 4,55 | 0,26 | 1,52 | 0,19 |
| Lauryl sulfate de sodium | 60,85 | 3,46 | 20,28 | 2,58 |
| HPMC 603 | 220,02 | 12,53 | 73,34 | 9,33 |
| Fénofibrate | 1161,66 | 66,14 | 387,22 | 49,23 |
| Talc | 8,74 | 0,50 | 3,91 | 0,50 |
| **Total** | 1756,48 | 100,00 | 786,49 | 100,00 |
| **Titre théorique (mg/g)** | 661,35 | | 492,34 | |

Le montage de la suspension de fénofibrate nanonisé est effectué en lit fluidisé GPCG1.

La lubrification s'est faite en turbine conventionnelle avec 0,5% de talc par rapport à la masse de granules produites.

Les principaux paramètres de montage et de séchage sont résumés dans le tableau 13.

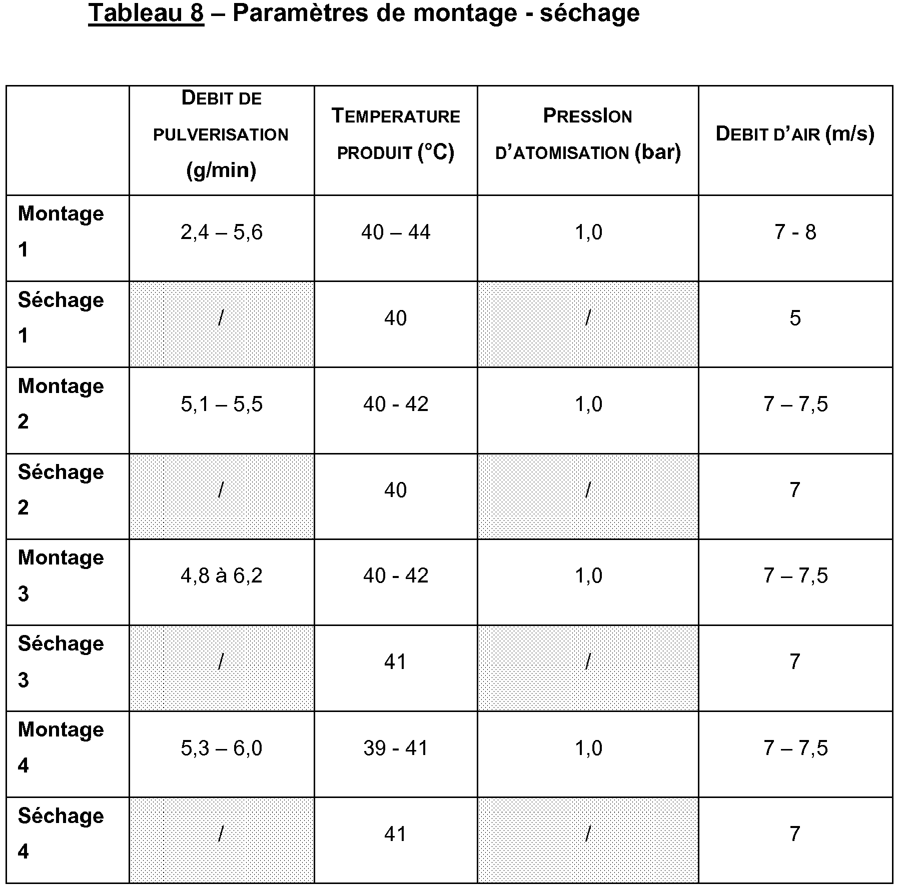

Les caractéristiques des granulés obtenus sont présentées dans le tableau 14.

**Tableau 9 - Caractéristiques des granulés**

| **LOT** | **4 MONTAGES** | **2 MONTAGES** |
|---|---|---|
| **RENDEMENT EN MASSE** | 90,9% | 91,8% |
| **TITRE THEORIQUE (mg/g)** | 661,35 | 492,34 |
| **Dissolution (voir** **figure 2****)** | N/E | 5 min : 41,5% |
| | | 10 min : 68,9% |
| | | 15 min : 88,6% |
| | | 30 min : 98,4% |
| | | 45 min : 98,4% |
| | | 60 min : 98,6% |

Les microgranules 2 montages sont mis en gélules gélatine #2 (teneur théorique en fénofibrate : 130mg).

### E- Préparation des gélules

Les granules 2 montages sont mis en gélules dosées à 130 mg. La teneur des granules en fénofibrate est de 475,9 mg/g. La masse de remplissage est donc de 273,2 mg de granules par gélule.

La masse moyenne des gélules pleines est de 337,1 mg.

**Tableau 10 - Résultats analytiques des gélules**

| **TEST** | | **RESULTATS** |
|---|---|---|
| | | 102,2% |
| | | 102,1% |
| | | 101,3% |
| | | 100,4% |
| | | 100,3% |
| | | 104,0% |
| Uniformité de teneur | | 100,4% |
| | | 102,7% |
| | | 101,6% |
| | | 104,9% |
| | | |
| | | X = 102,0% / SD = 1,5 / VA = 4,2 Conforme : niveau 1 |
| Dissolution (voir figure 3) | 5 min | 2,7% |
| | 10 min | 37,0% |
| | 15 min | 66,4% |
| | 20 min | 86,2% |
| | 25 min | 96,8% |
| | 30 min | 100,2% |
| | 45 min | 102,0% |
| | 60 min | 102,1% |
| Dosage en fénofibrate | 132,6 mg / gélule | |

### Exemple 3 : Préparation d'une formulation de fénofibrate nanonisé sous forme de comprimé

### A- Préparation de la suspension

a. La siméthicone est ajoutée dans l'eau sous agitation,
b. La diméthicone est ajoutée et maintenue sous agitation jusqu'à complète dissolution,
c. Le lauryl sulfate de sodium est ajouté et maintenu sous agitation jusqu'à complète dissolution,
d. L'HPMC 603 est ajoutée et maintenue sous agitation jusqu'à complète dissolution,
e. Le fénofibrate est ajouté et maintenu sous agitation jusqu'à complète dispersion,
f. L'agitation est maintenue jusqu'à complète dispersion,

Cette suspension est broyée dans un broyeur humide chargé de billes d'oxyde de zirconium de 0,2 mm (80%). Le broyage est réalisé à faible vitesse (de environ 1500 rpm à 2500 rpm). La température du produit est maintenue en dessous de 35°C.

### B- Formule de la suspension

**Tableau 11 - Formulation de la suspension**

| | **MASSE (G)** | **POURCENTAGE** |
|---|---|---|
| **Fénofibrate** | 387.5 | 22.73 |
| **HPMC 603** | 22.2 | 1.30 |
| **Diméthicone 35%** | 4.3 | 0.25 |
| **Siméthicone 30%** | 0.7 | 0.04 |
| **Laurylsulfate de Sodium** | 19.8 | 1.16 |
| **Eau** | 1270.1 | 74.51 |
| **Total** | 1704.6 | 100.00 |

### C- Préparation des granules

La suspension est montée sur des granules de sucre (400 - 600 µm) dans un FBC (GPCG1, Glatt, bottom spray, 1,2 mm), de sucrose (diamètre 540 µm), sur des granules de cellulose microcristalline (Ethispheres 100, Cellets 100, Avicel PH200), ou du lactose directement compressible.

Les granules de Lactose DLC21 sont retenus pour la fabrication des comprimés en raison de leurs propriétés favorables en compression et dissolution.

**Tableau 12 - Formule des granules sur Avicel PH200**

| | **MASSE (G)** | **POURCENTAGE (%)** |
|---|---|---|
| Lactose DCL21 | 700.00 | 62.83 |
| Fénofibrate | 326.01 | 29.26 |
| Siméthicone (dry) | 0.18 | 0.02 |
| Diméthicone (dry) | 1.27 | 0.11 |
| HPMC 603 | 61.62 | 5.53 |
| laurylsulfate de Sodium | 17.04 | 1.53 |
| Talc | 8.00 | 0.72 |
| eau | *1457.08** | /* |
| Total dry mass | 1114.12 | 100.00 |

| | | |
|---|---|---|
| * enlevé pendant l'étape de séchage | | |

### D- Préparation des comprimés

**Tableau 13 - Formulation des comprimés de Fénofibrate 145 mg**

| | **FORMULE PAR UNITE (MG)** | **POURCENTAGE (%)** |
|---|---|---|
| Lactose DCL21 | 449.8 | 69.14 |
| Fénofibrate | 144.9 | 22.27 |
| Siméthicone (dry) | 0.1 | 0.02 |
| Diméthicone (dry) | 0.6 | 0.09 |
| HPMC 603 | 27.4 | 4.21 |
| lauryl sulfate de Sodium | 7.6 | 1.17 |
| AcDiSol | 19.6 | 3.01 |
| Stéarate de Magnésium | 0.6 | 0.09 |
| **Total** | **650.6** | **100.00** |

Les granules à base de lactose sont comprimés avec du lactose comme diluant, de la croscarmellose sodique comme agent désintégrant et du stéarate de magnésium comme lubrifiant.

Les paramètres de compression sont les suivants:
- Machine de compression SVIAC PR12
- poinçons oblongs 15 mm x 7 mm
- masse moyenne: 654,1 mg/comprimé
- dureté moyenne: 74,4 N

### E- Analyse cristallographique en diffraction des rayons X

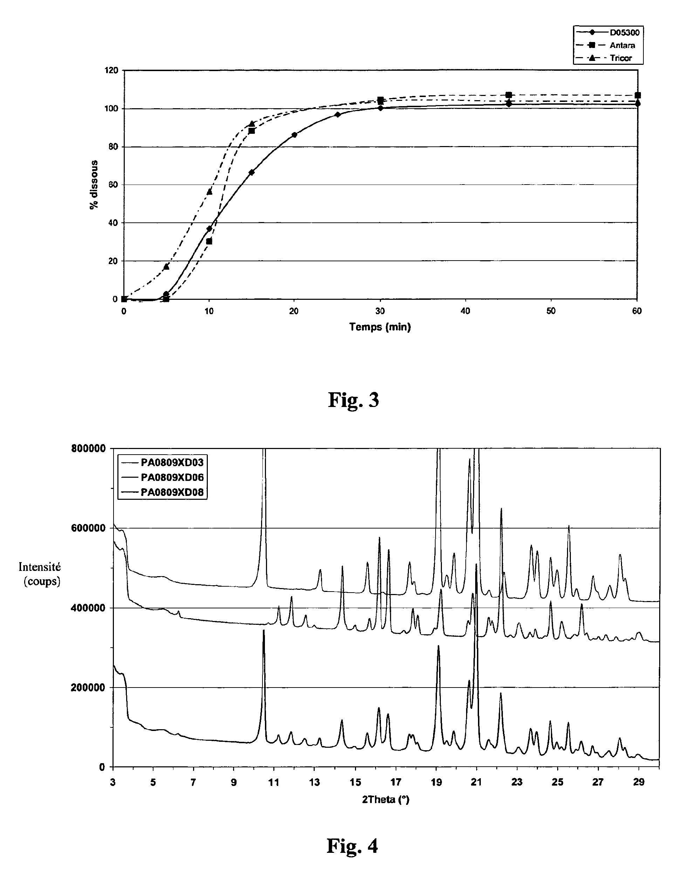

La figure 4 montre les diffractogrammes du fénofibrate (en haut), du lactose anhydre (au milieu) et des granules montés sur lactose anhydre (en bas). Tous les pics des granules peuvent être attribués au fénofibrate ou au lactose anhydre. Les étapes de broyage et de montage n'induisent pas d'apparition de fénofibrate amorphe.

La figure 5 montre les diffractogrammes des granules (en haut), et de deux lots de comprimés (au milieu et en bas). Tous les pics peuvent être attribués au fénofibrate ou au lactose anhydre. Ils ne comprennent donc pas de fénofibrate en phase amorphe.

### F- Analyses pharmacocinétiques

1-Comparaison du profil de libération *in vivo* des comprimés contenant les granules dosées à 145 mg de fénofibrate nanonisé réalisé chez le sujet à jeun, (TEST) à celui des comprimés commercialisés sous la marque Lipanthyl® (granules dosées à 145 mg de fénofibrate nanonisé), réalisé chez le sujet à jeun
Le produit Lipanthyl® est considéré comme le produit de référence.
Cette étude est réalisée chez 31 sujets. Des prélèvements sanguins sont réalisés à des intervalles de temps réguliers et l'acide fénofibrique est dosé. Les résultats sont présentés dans les tableaux 19 et 20.
Les abréviations suivantes sont utilisées dans la présente demande : Cmax : concentration plasmatique maximale exprimée en ng/mL,
Tmax : temps nécessaire pour atteindre le Cmax,
AUCt : aire sous la courbe de 0 à t, exprimée en ng.h/mL t représente le dernier temps avec concentration mesurable
AUC∞ : aire sous la courbe de 0 à l'infini, exprimée en ng.h/mL

Ces résultats montrent que la composition administrée dans les conditions du « TEST » est bioéquivalente au produit de référence Lipanthyl® administré chez le sujet à jeun.

**Tableau 19**

| **PARAMETRE** | **TEST** | | **REFERENCE** | |
|---|---|---|---|---|
| | **MOYENNE** | C.V. (%) | **MOYENNE** | C.V. (%) |
| Cmax (ng/mL) | 9416.8 | 20.3 | 10385.4 | 88.8 |
| tmax (hours) | 2.00 | 49.5 | 2.00 | 40.9 |
| AUCt (ng·h/mL) | 158561.9 | 53.6 | 158012.6 | 39.2 |
| AUCα (ng·h/mL) | 169277.6 | 66.1 | 163551.1 | 41.0 |
| AUCt/α (%) | 96.46 | 5.0 | 97.19 | 2.2 |

**Tableau 20**

| **PARAMETRE** | **TEST** | | **REFERENCE** | |
|---|---|---|---|---|
| | **MOYENNE** | C.V. (%) | **MOYENNE** | C.V. (%) |
| Cmax (ng/mL) | 9416.8 | 20.3 | 10385.4 | 18.8 |
| tmax (hours) | 2.00 | 49.5 | 2.00 | 40.9 |
| AUCt (ng·h/mL) | 158561.9 | 53.6 | 158012.6 | 39.2 |
| AUCα (ng·h/mL) | 169277.6 | 66.1 | 163551.1 | 41.0 |
| AUCt/α (%) | 96.46 | 5.0 | 97.19 | 2.2 |

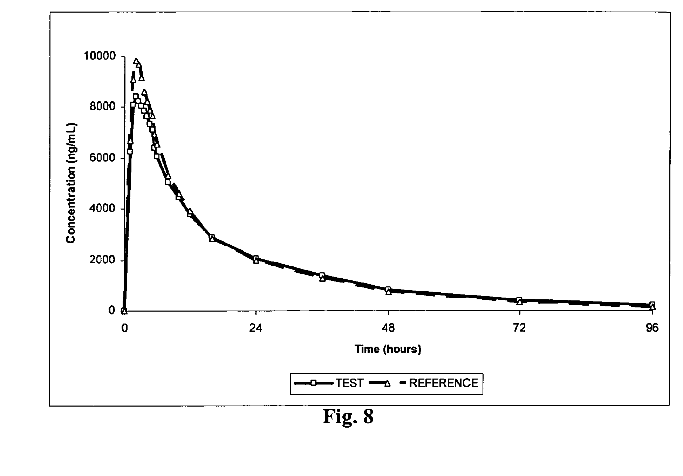

Les résultats sont représentés graphiquement à la Figure 8.
2-Comparaison du profil de libération *in vivo* des comprimés contenant les granules dosées à 145 mg de fénofibrate nanonisé réalisé chez le sujet à jeun, (TEST) à celui des comprimés commercialisés sous la marque Lipidil EZ® (granules dosées à 145 mg de fénofibrate nanonisé), réalisé chez le sujet à jeun
Le produit Lipidil EZ® est considéré comme le produit de référence.
Cette étude est réalisée chez 19 sujets. Des prélèvements sanguins sont réalisés à des intervalles de temps réguliers et l'acide fénofibrique est dosé. Les résultats sont présentés dans les tableaux 21 et 22.

**Tableau 21**

| **PARAMETRE** | **TEST** | **REFERENCE** |
|---|---|---|
| Cmax (nq/mL) | **10615.4** (26.2) | **10920.6** (21.3) |
| tmax (hours) | **2.00** [1-3.53] | **2.00** [1.00-5.00] |
| AUCt* | **173297.4** (38.2) | **167147.5** (35.1) |
| (ng·h/mL) | | |
| AUCα* (ng.h/mL) | **179028.2** (38.5) | **171952.5** (35.8) |
| AUCt/α* (%) | **96.92** (2.1) | **97.39** (1.5) |
| t1/2el* (h) | **19.09** (26.08) | **18.18** (23.64) |

**Tableau 22**

| **Paramètre** | **Ratio (%)** | **IC90%** |
|---|---|---|
| **Cmax** | **96.13** | **X** |
| **AUCt*** | **102.94** | **[98.61 - 107.46]** |
| **AUCα*** | **103.41** | **[99.25 - 107.75]** |

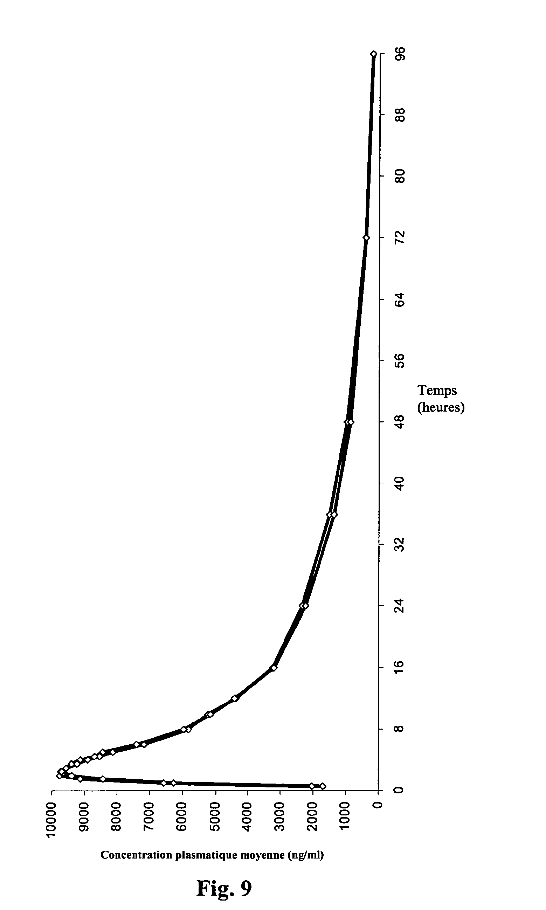

Ces résultats montrent que la composition administrée dans les conditions du « TEST » est bioéquivalente au produit de référence Lipidil EZ® administré chez le sujet à jeun.
Les résultats sont représentés graphiquement à la Figure 9.

### Exemple 4 : Préparation d'une formulation de fénofibrate nanonisé sous forme de gélule comprenant 0,3% de lécithine de soja dans la suspension

### A- Préparation de la suspension

Préparation de la suspension à broyer :
- introduire l'eau dans un récipient de taille adaptée,
- ajouter la suspension de siméthicone sous agitation constante,
- ajouter l'HPMC603 sous agitation,
- ajouter le laurylsulfate de sodium sous agitation constante, poursuivre l'agitation jusqu'à parfaite homogénéisation,
- ajouter la lécithine de soja sous agitation constante, poursuivre l'agitation jusqu'à parfaite homogénéisation,
- ajouter le fénofibrate sous agitation constante, poursuivre l'agitation pendant au moins 30 minutes avant transfert de la suspension dans le broyeur, maintenir l'agitation pendant le broyage.

La chambre (1,1 L) du broyeur HOSOKAWA 90AHM est chargée à 80% avec des billes de polystyrène de diamètre 0,36-0,5 mm, Le broyeur est utilisé en mode recirculation. La puissance de broyage est adaptée pour atteindre la granulométrie visée (d(50) < 250nm).

Le système est refroidi en continu pour que la température produit ne dépasse pas 45°C.

L'efficacité du broyage est vérifiée par mesure de la granulométrie (Mastersizer Malvern 2000hydroS).

Les paramètres de broyage sont les suivants :
Vitesse de pompe : 100 rpm
Vitesse de broyage: 2500 rpm
Temps de broyage total : 300 min

Le tableau 14 présente la formule quantitative (g) et centésimale (%) de la suspension prête à pulvériser.

**Tableau 14 : Quantités de matières par suspension de broyage**

| **Matière** | **FORMULE QUANTITATIVE (G)** | **FORMULE CENTESIMALE(%)** |
|---|---|---|
| **Eau purifiée** | **3652,30** | **78,01** |
| **Fénofibrate** | **817,11** | **17,45** |
| **HPMC 603** | **154,67** | **3,30** |
| **Siméthicone (em.30%)** | **1,54** | **0,03** |
| **Lauryl sulfate de sodium** | **42,76** | **0,91** |
| **Lécithine de soja** | **13,62** | **0,29** |
| **Total** | **4682,00** | **100,00** |

### B- Préparation des microgranules

Le tableau 15 présente la formule centésimale et quantitative des granules de fénofibrate.

**Tableau 15: Formule des granules de fénofibrate**

| **Matière** | **Formule quantitative (g)** | **Formule centésimale (%)** |
|---|---|---|
| **Neutres 30** | 290,29 | 16,33 |
| **Fénofibrate** | 1119,84 | 62,99 |
| **Lécithine de Soja** | 18,66 | 1,05 |
| **Emulsion de siméthicone 30% (masse sèche)** | 0,63 | 0,04 |
| **HPMC 603** | 211,97 | 11,92 |
| **Laurylsulfate de sodium** | 58,61 | 3,30 |
| **Talc** | 31,79 | 1,79 |
| **HPMC 606** | 45,90 | 2,58 |
| **Eau purifiée** | - | * |
| **Total** | 1777,69 | 100,00 |

| | | |
|---|---|---|
| *solvant évaporé pendant le procédé | | |

Le procédé de fabrication consiste en :
(1) broyage du fénofibrate en suspension dans eau + siméthicone + laurylsulfate de sodium+ lécithine de soja +HPMC 603
(2) pulvérisation sur support neutre
(3) Surenrobage avec HPMC 606/talc

Le montage est réalisé en lit fluidisé pour obtenir une teneur en fénofibrate des microgranules supérieure à 600mg/g.

Les paramètres de montage sont les suivants :

| **Etapes** | **Paramètres** |
|---|---|
| Montage | Température air entrée : 50-60°C |
| | Température produit : 35-45°C |
| | Pression d'atomisation : 1-1,5 bar |
| | Débit d'air : 4-8 m/s |
| Séchage | Température air entrée : 50°C |
| | Température produit : 40°C |
| | Temps de séchage: 10 min |

Environ 1700 g de granules montés sont récupérés. Ils sont surenrobés à l'HPMC606/talc.

La formule quantitative et centésimale de la suspension d'enrobage est la suivante :
Les paramètres d'enrobage sont les suivants :

| **Etapes** | **Paramètres** |
|---|---|
| Montage | Température air entrée : 50-60°C |
| | Température produit: 35-45°C |
| | Pression d'atomisation : 1-1,5 bar |
| | Débit d'air : 4-8 m/s |
| | Température air entrée : 50°C |
| Séchage | Température produit : 40°C |
| | Temps de séchage: 10 min |

Les granules montés et surenrobés sont lubrifiés avec 0,5% de talc par rapport à la masse totale.

### C- Préparation des gélules

Les granules de l'étape B sont mis en gélules dosées à 130 mg.

### Exemple 5: Etudes pharmacocinétiques de la formulation de l'Exemple 4C

A- Comparaison du profil de libération *in vivo* des gélules contenant les granules dosées à 130 mg de fénofibrate nanonisé et comprenant de la lécithine de soja, réalisé chez le sujet à jeun, (Test 1) à celui des gélules commercialisées sous la marque Antara® (granules dosées à 130 mg de fénofibrate micronisé), réalisé chez le sujet venant de s'alimenter avec un repas pauvre en graisses
Le produit Antara® est considéré comme le produit de référence.

Cette étude est réalisée chez 12 sujets. Des prélèvements sanguins sont réalisés à des intervalles de temps réguliers et l'acide fénofibrique est dosé. Les résultats sont présentés dans le tableau 16.

**Tableau 16**

| Paramètres | Test1 | Référence | Ratio | Intervalle de confiance 90% | |
|---|---|---|---|---|---|
| Cmax | 6784.9 | 6767.4 | 100.26 | 91.18 | 110.24 |
| AUCt | 122904.6 | 118176.1 | 104.00 | 98.32 | 110.01 |
| AUC∞ | 127668.3 | 123349.7 | 103.50 | 97.96 | 109.36 |

Les abréviations suivantes sont utilisées dans la présente demande :
Cmax : concentration plasmatique maximale exprimée en ng/mL,
Tmax : temps nécessaire pour atteindre le Cmax,
AUCt : aire sous la courbe de 0 à t, exprimée en ng.h/mL
t représente le dernier temps avec concentration mesurable
AUC∞ : aire sous la courbe de 0 à l'infini, exprimée en ng.h/mL

Ces résultats montrent que la composition administrée dans les conditions du « Test1 » est bioéquivalente au produit de référence Antara® administré chez le sujet venant de s'alimenter avec un repas pauvre en graisses. La réduction de la taille des particules de fénofibrate combinée à l'ajout de lécithine de soja a amélioré la biodisponibilité du produit.

B- Comparaison du profil de libération *in vivo* des gélules contenant les granules dosées à 130 mg de fénofibrate nanonisé et comprenant de la lécithine de soja, réalisé chez le sujet venant de s'alimenter avec un repas pauvre en graisses (Test 2), à celui des gélules commercialisées sous la marque Antara® (granules dosées à 130 mg de fénofibrate micronisé), réalisé chez le sujet venant de s'alimenter avec un repas pauvre en graisses

Cette étude est réalisée chez 11 et 12 sujets respectivement. Des prélèvements sanguins sont réalisés à des intervalles de temps réguliers et on dose l'acide fénofibrique.

Les résultats sont présentés dans le tableau 17.

**Tableau 17**

| Paramètres | Test2 | Référence | Ratio | Intervalle de confiance 90% | |
|---|---|---|---|---|---|
| Cmax | 7333.8 | 6767.4 | 108.37 | 98.25 | 119.53 |
| AUCt | 123093.9 | 118176.1 | 104.16 | 98.27 | 110.41 |
| AUC∞ | 127435.2 | 123349.7 | 103.31 | 97.59 | 109.38 |

Ces résultats montrent que la composition administrée dans les conditions du « Test2 » est bioéquivalente au produit de référence Antara administré dans les mêmes conditions.

C- Comparaison du profil de libération *in* *vivo* des gélules contenant les granules dosées à 130 mg de fénofibrate **nanonisé** et comprenant de la lécithine de soja, réalisé chez le sujet venant de s'alimenter avec un repas pauvre en graisses (Test 2), à celui des gélules contenant les granules dosées à 130 mg de fénofibrate **nanonisé** et comprenant de la lécithine de soja, réalisé chez le sujet à jeun (Test 1).

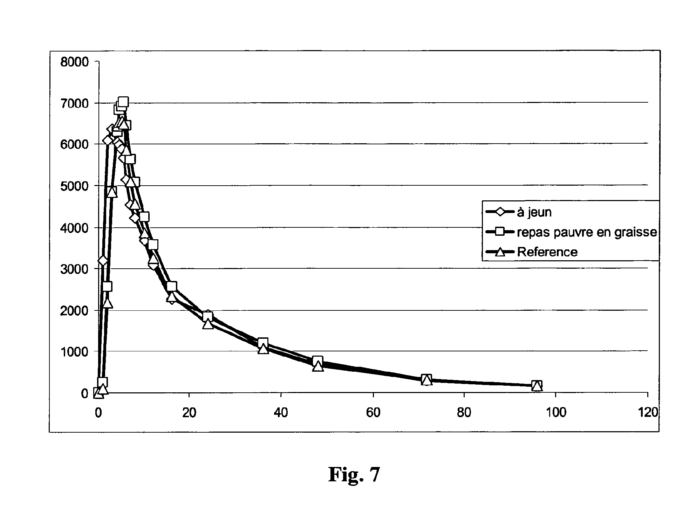

Les résultats sont présentés dans le tableau 18 et la Figure 7.

**Tableau 18**

| Paramètres | Test1 | Test2 | Ratio | Intervalle de confiance 90% | |
|---|---|---|---|---|---|
| Cmax | 6784.9 | 7333.8 | 92.52 | 83.88 | 102.04 |
| AUCt | 122904.6 | 123093.9 | 99.85 | 94.20 | 105.83 |
| AUC∞ | 127668.3 | 127435.2 | 100.18 | 94.63 | 106.06 |

Ces résultats montrent que la composition de gélules comprenant des granules de fénofibrate nanonisé et de la lécithine de soja est bioéquivalente dans les deux conditions d'administration, c'est-à-dire à jeun et avec un repas faiblement gras.

## Revendications

1. Procédé de préparation d'une suspension aqueuse contenant du fénofibrate et/ou de l'acide fénofibrique en suspension, un dérivé cellulosique en tant qu'adjuvant de solubilisation et un tensioactif, **caractérisée en ce que** le fénofibrate et/ou l'acide fénofibrique sont sous forme cristalline, présentant respectivement un point de fusion en DSC de 79°C à 82°C ou de 179°C à 183°C, et **en ce que** la taille moyenne des particules de fénofibrate ou d'acide fénofibrique (D(50)) est inférieure à 250 nm, de préférence 180 nm, **caractérisé par** les étapes suivantes :
(a) mélanger une partie de la proportion de dérivé cellulosique dans la suspension finale, le tensioactif et éventuellement le phospholipide avec de l'eau sous agitation jusqu'à complète dissolution,
(b) ajouter le fénofibrate et/ou l'acide fénofibrique au mélange obtenu à l'étape précédente sous agitation jusqu'à complète dissolution, puis ;
(c) broyer le mélange obtenu à l'étape précédente en présence de billes, de préférence de billes de polymère ou d'oxyde de zirconium et/ou d'yttrium, à une puissance suffisante pour atteindre une granulométrie moyenne du fénofibrate (D(50)) inférieure à 250 nm ;
(d) éventuellement homogénéiser la suspension broyée obtenue à l'étape précédente ;
(e) ajuster la quantité de dérivé cellulosique et éventuellement d'eau,
et **caractérisé en ce que** le dérivé cellulosique est l'hydroxypropylméthylcellulose.

2. Procédé selon la revendication 1, **caractérisée en ce que** la taille de 90% des particules (D(90)) de fénofibrate est inférieure à 500 nm, de préférence 400 nm.

3. Procédé selon la revendication 1 ou 2, comprenant en outre un phospholipide, préférentiellement la lécithine de soja.

4. Procédé selon la revendication 3, **caractérisée en ce que** la proportion de phospholipide est comprise entre 0,25% et 10%, de préférence entre 0,3 et 5%, en poids de la suspension.

5. Procédé de préparation de microgranules ou de granules de fénofibrate et/ou d'acide fénofibrique comprenant une étape (a) de pulvérisation d'une ou plusieurs couches, avantageusement deux, d'une suspension obtenue par le procédé selon l'une quelconque des revendications 1 à 4 sur des neutres ou des granules d'excipient.

6. Microgranules ou granules de fénofibrate et/ou d'acide fénofibrique susceptibles d'être obtenus par un procédé selon la revendication 5.

7. Microgranules ou granules de fénofibrate et/ou d'acide fénofibrique selon la revendication 6, **caractérisés en ce que** la proportion de phospholipide est comprise entre 1% et 10%, de préférence entre 3 et 6%, en poids de microgranules.

8. Microgranules ou granules de fénofibrate et/ou d'acide fénofibrique selon la revendication 6 ou 7, **caractérisés en ce que** la proportion de fénofibrate est supérieure à 60% en poids de microgranules.

9. Microgranules ou granules de fénofibrate et/ou d'acide fénofibrique selon l'une quelconque des revendications 6 à 8, **caractérisés en ce que** la proportion de tensioactif est comprise entre 1% et 10%, de préférence entre 3 et 5%, en poids de microgranules.

10. Microgranules ou granules de fénofibrate et/ou d'acide fénofibrique selon l'une quelconque des revendications 6 à 9, **caractérisés en ce que** la proportion d'hydroxypropylméthylcellulose en tant que liant et adjuvant de solubilisation est comprise entre 7% et 20%, de préférence entre 10 et 15%, en poids de microgranules.

11. Formulation pharmaceutique unitaire solide comprenant des microgranules ou des granules selon l'une quelconque des revendications 6 à 10, comprenant entre 20 et 200 mg de fénofibrate, de préférence entre 110 et 145 mg de fénofibrate.

12. Formulation pharmaceutique unitaire solide comprenant des microgranules ou des granules selon l'une quelconque des revendications 6 à 10, comprenant entre 18 et 180 mg d'acide fénofibrique.

13. Formulation pharmaceutique selon la revendication 11, comprenant entre 110 et 120 mg de fénofibrate, ou entre 30 et 40mg de fénofibrate.

14. Formulation pharmaceutique de fénofibrate et/ou d'acide fénofibrique selon l'une quelconque des revendications 11 à 13 pour le traitement des hypertriglycéridémies, hypercholestérolémies ou hyperlipidémies.

15. Formulation pharmaceutique de fénofibrate et/ou d'acide fénofibrique selon la revendication 14, le profil pharmacocinétique du fénofibrate étant équivalent que le patient soit nourri avec un repas à forte teneur en matières grasses ou à jeun.

## Patentansprüche

1. Herstellungsverfahren von einer Wässrige Suspension, enthaltend Fenofibrat und/oder Fenofibratsäure in Suspension, ein Zellulosederivat als Solubilisierungsadjuvans und ein Tensid, **dadurch gekennzeichnet, dass** das Fenofibrat und/oder die Fenofibratsäure in kristalliner Form sind, aufweisend jeweils einen Schmelzpunkt in DSC von 79 °C bis 82 °C oder von 179 °C bis 183 °C, und dass die durchschnittliche Größe der Fenofibrat- oder Fenofibratsäurepartikel (D(50)) unter 250 nm, vorzugsweise 180 nm, ist, **gekennzeichnet durch** die folgenden Schritte:
(a) Mischen eines Teils des Anteils von Zellulosederivat in der finalen Suspension, das Tensid und eventuell das Phospholipid mit Wasser unter Rühren bis zur vollständigen Auflösung,
(b) Hinzufügen des Fenofibrats und/oder der Fenofibratsäure zu dem in vorangegangenem Schritt hergestellten Gemisch unter Rühren bis zur vollständigen Auflösung, dann
(c) Zerkleinern des in dem vorangegangenen Schritt hergestellten Gemischs bei Anwesenheit von Kugeln, vorzugsweise von Polymer- oder Zirkonium- und/oder Yttriumoxidkugeln mit einer Leistung, die ausreichend ist, um eine durchschnittliche Korngröße des Fenofibrats (D(50)) unter 250 nm zu erreichen,
(d) eventuell Homogenisieren der zerkleinerten, in vorangegangenem Schritt hergestellten Suspension,
(e) Korrigieren der Zellulosederivat- und eventuell der Wassermenge,
und **dadurch** gekennzeichnet, dass das Zellulosederivat die Hydroxypropylmethylzellulose ist.

2. Verfahren nach Anspruch 1, **dadurch gekennzeichnet, dass** die Größe von 90 % der Fenofibratpartikel (D(90)) unter 500 nm, vorzugsweise 400 nm, ist.

3. Verfahren nach Anspruch 1 oder 2, umfassend ferner ein Phospholipid, vorzugsweise Sojalecithin.

4. Verfahren nach Anspruch 3, **dadurch gekennzeichnet, dass** der Phospholipidanteil zwischen 0,25 Gew.-% und 10 Gew.-%, vorzugsweise zwischen 0,3 und 5 Gew.-%, inklusive der Suspension ist.

5. Herstellungsverfahren von Fenofibrat- und/oder Fenofibratsäure-Mikrogranulat oder -Granulat, umfassend einen Pulverisierungsschritt (a) einer oder mehrerer, vorzugsweise von zwei, Schichten einer Suspension nach einem Verfahren der Ansprüche 1 bis 4 auf neutralem oder Exzipientgranulat.

6. Fenofibrat- und/oder Fenofibratsäure-Mikrogranulat oder -Granulat, das mit einem Verfahren nach Anspruch 5 herstellbar ist.

7. Fenofibrat- und/oder Fenofibratsäure-Mikrogranulat oder -Granulat nach Anspruch 6, **dadurch gekennzeichnet, dass** der Anteil des Phospholipids zwischen 1 Gew.-% und 10-Gew.-%, vorzugsweise zwischen 3 und 6 Gew.-%, inklusive des Mikrogranulats ist.

8. Fenofibrat- und/oder Fenofibratsäure-Mikrogranulat oder -Granulat nach Anspruch 6 oder 7, **dadurch gekennzeichnet, dass** der Anteil des Fenofibrats über 60 Gew.-% des Mikrogranulats ist.

9. Fenofibrat- und/oder Fenofibratsäure-Mikrogranulat oder -Granulat nach einem der Ansprüche 6 bis 8, **dadurch gekennzeichnet, dass** der Anteil des Tensids zwischen 1 Gew.-% und 10 Gew.-%, vorzugsweise zwischen 3 und 5 Gew.-%, inklusive des Mikrogranulats ist.

10. Fenofibrat- und/oder Fenofibratsäure-Mikrogranulat oder -Granulat nach einem der Ansprüche 6 bis 9, **dadurch gekennzeichnet, dass** der Anteil der Hydroxypropylmethylzellulose als Bindemittel und Solubilisierungsadjuvans zwischen 7 Gew.-% und 20 Gew.-%, vorzugsweise zwischen 10 und 15 Gew.-%, inklusive des Mikrogranulats ist.

11. Feste einheitliche pharmazeutische Formulierung, umfassend Mikrogranulat oder Granulat nach einem der Ansprüche 6 bis 10, umfassend zwischen 20 und 200 mg Fenofibrat, vorzugsweise zwischen 110 und 145 mg Fenofibrat.

12. Feste einheitliche pharmazeutische Formulierung, umfassend Mikrogranulat oder Granulat nach einem der Ansprüche 6 bis 10, umfassend zwischen 18 und 180 mg Fenofibratsäure.

13. Pharmazeutische Formulierung nach Anspruch 11, umfassend zwischen 110 und 120 mg Fenofibrat oder zwischen 30 und 40 mg Fenofibrat.

14. Pharmazeutische Fenofibrat- und/oder Fenofibratsäureformulierung nach einem der Ansprüche 11 bis 13 für die Behandlung von Hypertriglyceridämien, Hypercholesterolämien oder Hyperlipidämien.

15. Pharmazeutische Fenofibrat- und/oder Fenofibratsäureformulierung nach Anspruch 14, wobei das pharmazeutische Profil des Fenofibrats äquivalent ist, unabhängig davon, ob der Patient mit einer Mahlzeit mit hohem Fettgehalt ernährt wird oder nüchtern ist.

## Claims

1. A method for preparing a suspension containing fenofibrate and/or fenofibric acid in suspension, a cellulose derivative as a solubilization adjuvant and a surfactant, **characterized in that** the fenofibrate and/or the fenofibric acid are in crystalline form, with a melting point in DSC of 79°C to 82°C or 179°C to 183°C, respectively, and **in that** the average size of the particles (D50) of fenofibrate or of fenofibric acid is less than 250 nm, preferably 180 nm, **characterized by** the following steps:
(a) mixing part of the proportion of cellulose derivative in the final suspension, the surfactant and, optionally, the phospholipid with water under agitation until complete dispersion;
(b) adding the fenofibrate and/or the fenofibric acid to the mixture obtained in the preceding step under agitation until complete dispersion; then,
(c) grinding the mixture obtained in the preceding step in the presence of beads, preferably beads of polymer or of zirconium and/or yttrium oxide, at a power sufficient to achieve an average particle size (D50) of the fenofibrate of less than 250 nm;
(d) optionally, homogenizing the ground suspension obtained in the preceding step;
(e) adjusting the quantity of cellulose derivative and, optionally, of water,
and **characterized in that** the cellulose derivative is hydroxypropyl methylcellulose.

2. The method according to claim 1, **characterized in that** the size of 90% of the particles (D90) of fenofibrate is less than 500 nm, preferably 400 nm.

3. The method according to claim 1 or 2, further comprising a phospholipid, preferentially soy lecithin.

4. The method according to claim 3, **characterized in that** the proportion of phospholipid is between 0.25% and 10%, preferably between 0.3% and 5%, by weight of the suspension.

5. A method for preparing microgranules or granules of fenofibrate and/or fenofibric acid, comprising a step (a) of the spraying of one or more layers, advantageously two, of a suspension obtained according to any one of claims 1 to 4 on neutrals or on granules of excipient.

6. The microgranules or granules of fenofibrate and/or fenofibric acid which can be obtained by the method according to claim 5.

7. The microgranules or granules of fenofibrate and/or fenofibric acid according to claim 6, **characterized in that** the proportion of the phospholipid is between 1% and 10%, preferably between 3% and 6%, by weight of the microgranules.

8. The microgranules or granules of fenofibrate and/or fenofibric acid according to claim 6 or 7, **characterized in that** the proportion of fenofibrate is greater than 60% by weight of the microgranules.

9. The microgranules or granules of fenofibrate and/or fenofibric acid according to any one of claims 6 to 8, **characterized in that** the proportion of surfactant is between 1% and 10%, preferably between 3% and 5%, by weight of the microgranules.

10. The microgranules or granules of fenofibrate and/or fenofibric acid according to any one of claims 6 to 9, **characterized in that** the proportion of hydroxypropyl methylcellulose as a binder and a solubilization adjuvant is between 7% and 20%, preferably between 10% and 15%, by weight of the microgranules.

11. A solid dosage pharmaceutical formulation comprising the microgranules or granules according to any one of claims 6 to 10, comprising between 20 mg and 200 mg of fenofibrate, preferably between 110 mg and 145 mg of fenofibrate.

12. The solid dosage pharmaceutical formulation comprising the microgranules or granules according to any one of claims 6 to 10, comprising between 18 mg and 180 mg of fenofibric acid.

13. The pharmaceutical formulation according to claim 11, comprising between 110 mg and 120 mg of fenofibrate, or between 30 mg and 40 mg of fenofibrate.

14. The pharmaceutical formulation of fenofibrate and/or fenofibric acid according to any one of claims 11 to 13, for the treatment of hypertriglyceridemia, hypercholesterolemia or hyperlipidemia.

15. The pharmaceutical formulation of fenofibrate and/or fenofibric acid according to claim 14, **characterized in that** the pharmacokinetic profile of the fenofibrate is equivalent whether the patient has consumed a high-fat meal or has fasted.
